# EUROPEAN PATENT APPLICATION

(11) **EP 1 470 831 A1**
(43) Date of publication of application: **27.10.2004**
(21) Application number: 04009724.8
(22) Date of filing: 23.04.2004
(51) Int. Cl.: A61L 31/16, A61M 25/00, A61F 2/06

(54) **Coated stent with protective packaging and method of using same**

(30) Priority: 23.04.2003 US 464865 P
(71) Applicant: Medtronic Vascular, Inc., Santa Rosa, California 95403 (US)
(72) Inventor: Farrell, Thomas c/o Medtronic Vascular, Inc., Galway (IE); Colm, Quinn c/o Medtronic Vascular, Inc., Galway (IE)
(74) Representative: Hughes, Andrea Michelle

(57) **Abstract**

A coated stent with protective packaging is provided. The coated stent comprises at least one stent segment 20and a tray 90 including a stent void 22 disposed therein, wherein the stent segment is restricted from movement with respect to the tray while disposed within the stent void. Systems and method for the coated stent are also provided.

## Description

### FIELD OF THE INVENTION

The invention relates to cardiovascular stents, and specifically to a coated stent with protective packaging.

### BACKGROUND OF THE INVENTION

Cardiovascular disease, including atherosclerosis, is the leading cause of death in the U.S. A number of methods and devices for treating coronary heart disease have been developed, some of which are specifically designed to treat the complications resulting from atherosclerosis and other forms of coronary arterial narrowing.

One method for treating atherosclerosis and other forms of coronary narrowing is percutaneous transluminal coronary angioplasty, hereinafter referred to as "angioplasty" or "PTCA". More than one-third of heart disease patients undergo angioplasty, approximating 1 million people annually worldwide. Some patients undergo angioplasty repeatedly.

The objective in angioplasty is to enlarge the lumen of the affected coronary artery by radial hydraulic expansion. This is generally accomplished by inflating a balloon within the narrowed lumen of the affected artery. Radial expansion of the coronary artery may occur in several different dimensions, and is related to the nature of the plaque. Soft, fatty plaque deposits are flattened by the balloon, while hardened deposits are cracked and split to enlarge the lumen. The wall of the artery itself may also be stretched as the balloon is inflated.

With simple angioplasty, the balloon is threaded through the artery with a catheter and inflated at the place where the blood vessel is blocked. After the procedure, the balloon is then removed. With simple angioplasty alone, about 40-50 percent of arteries close up again or re-narrow. This narrowing is known as restenosis.

To reduce the risk of restenosis, a stent may also be inserted during angioplasty. The stent may be used to prop open the artery once the balloon is removed. The use of a stent may reduce the risk of restenosis to 20-30 percent. The stent is designed to support plaque damaged arterial walls after a blockage has been removed.

Typically, if restenosis occurs with a stent, the doctors may insert highly radioactive pellets into the artery to help prevent further clogging. This radiation therapy can halve the risk of restenosis but presents all the risks associated with radiation therapy.

Restenosis occurs because the blood vessel wall is injured when the stent is implanted. The area then becomes inflamed and new cells form scar tissue. The arterial walls may become so thick in some instances that they protrude into the mesh of the stent. In such cases, a further angioplasty may be undergone, and a new stent may be placed inside the existing one. If restenosis continues, the eventual alternative may then be bypass surgery.

Alternatively, a coated stent may be inserted during the angioplasty. Such a coated stent may eliminate the need for repeat angioplasties and could spare some patients the trauma, risk and prolonged recovery associated with heart bypass surgery.

The coated stent may be coated, for example, with Rapamune, generically known as sirolimus or rapamycin. This drug is used to prevent organ rejection in kidney transplants. It stops new cells from forming without impairing the healing of the vessel. It also dampens inflammation and has antibiotic properties.

In clinical studies, patients who have received the coated stent do not show this re-narrowing and re-blockage of arteries.

However, because the coating of the stent comprises a therapeutic drug, coated stents present problems associated with drug administration. For example, for a drug to be administered effectively, the integrity of the drug's effective dosage should be maintained. Additionally, contamination of the drug should be avoided. Certain drugs also require regulated conditions for efficacy, such as regulated air circulation or lack thereof, regulated exposure to light, etc. Furthermore, some processes in preparing the stent for use, such as sterilization, may affect the drug's efficacy.

Currently, stents are packaged in a sterile flat tray. The stent and balloon catheter are coiled and the stent is secured between the tray and a peel away top. With a coated stent, the tray may damage the coating while the stent is being placed on the tray. Meanwhile, the peel away top may stick to the coating and when the top is removed, the coating may be removed with the top rather than remaining on the stent. Additionally, the stent may move within the package during handling. With such movement, some of the coating may be disturbed. In any of these cases, the coating of the stent may be damaged and the dosage of the drug may be reduced.

Moreover, current packaging for stents does not provide for regulation of ambient conditions such as circulation of air or exposure to light. Without such appropriate regulation, the efficacy of the drug may be reduced.

It would be desirable therefore to provide a packaging assembly for a coated stent that overcomes the above.

### SUMMARY OF THE INVENTION

One aspect of the present invention provides a coated stent with protective packaging. The coated stent comprises at least one stent segment and a tray including a stent void disposed therein, wherein the stent segment is restricted from movement with respect to the tray while disposed within the stent void. The coated stent may also comprise a stylet disposed within the tray proximate the stent void to suspend the at least one stent segment within the stent void. In addition, the stent may comprise at least one first attachment disposed within the tray distal to the stent void, to retain the stent segment within the stent void and at least one second attachment disposed within the tray proximal to the stent void to retain the stent segment within the stent void. In some embodiments of the invention, a coating may be dispersed on the at least one stent segment, and the coating may be any one or more of the following: thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives, anticancer chemotherapeutic agents, anti-inflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, inhibitors, free radical scavengers, chelators, antioxidants, antipolymerases, antiviral agents, photodynamic therapy agents, gene therapy agents, and conjugates thereof.

The coated stent may also comprise a catheter attached to the stent segment, an expandable balloon portion attached to the balloon catheter, wherein the expandable balloon portion expands an inner lumen of the stent segment and a catheter void disposed within the tray, wherein the catheter void holds the catheter. In some embodiments of the invention, the stent may also comprise at least one catheter attachment disposed proximate the catheter void, wherein the catheter attachment retains the catheter within the catheter void.

The coated stent may also include an introducer to receive the stent segment and an introducer void disposed within the tray to hold the introducer.

In addition, the coated stent may include at least one accessory void disposed within the tray to hold an accessory such as desiccant packets, oxygen absorber packets, chemical packets, catheters, guidewires, cannulas and stylets. The coated stent may also include a lid.

Another aspect of the present invention provides a system for treating heart disease comprising a stent coupled to a catheter, the stent including a drug polymer coating and a tray including a stent void disposed therein, wherein the stent is restricted from movement in relation to the tray when the stent is placed in the stent void. The system may also include a stylet disposed proximate the stent void, wherein the stylet suspends the stent within the stent void.

In addition, the system may include at least one first attachment disposed distal to the stent void to retain the stent segment within the stent void and at least one second attachment disposed proximal to the stent void, wherein the second attachment retains the stent segment within the stent void.

In some embodiments of the invention, system includes an expandable balloon portion attached to the catheter and may also include a catheter void to hold the catheter within the tray. The system may also include an introducer to receive the stent segment and an introducer void to hold an introducer within the tray. The system may further include an accessory such as desiccant packets, oxygen absorber packets, chemical packets, catheters, guidewires, cannulas and stylets and at least one accessory void to hold at least one accessory within the tray. The system may also include a lid to seal the tray.

Another aspect of the present invention provides a method of protecting a coated stent. A tray is provided, including a stent void therein. The coated stent is suspended within the stent void such that the coated stent is restricted from movement with respect to the tray when placed in the stent void.

The foregoing, and other, features and advantages of the invention will become further apparent from the following detailed description of the presently preferred embodiments, read in conjunction with the accompanying drawings. The detailed description and drawings are merely illustrative of the invention rather than limiting, the scope of the invention being defined by the appended claims.

### BRIEF DESCRIPTION OF THE DRAWINGS

**FIG. 1** is a schematic view of one embodiment of a coated stent with protective packaging in accordance with the present invention;
**FIG. 2** is a cross-section of the embodiment of the coated stent with protective packaging of **FIG.1;**
**FIG. 3** is a schematic view of one embodiment of a lid for the coated stent with protective packaging system of **FIG. 1**; and
**FIG. 4** is a schematic view of one embodiment of a coated stent in accordance with the present invention.

### DETAILED DESCRIPTION OF THE PRESENTLY PREFERRED EMBODIMENTS

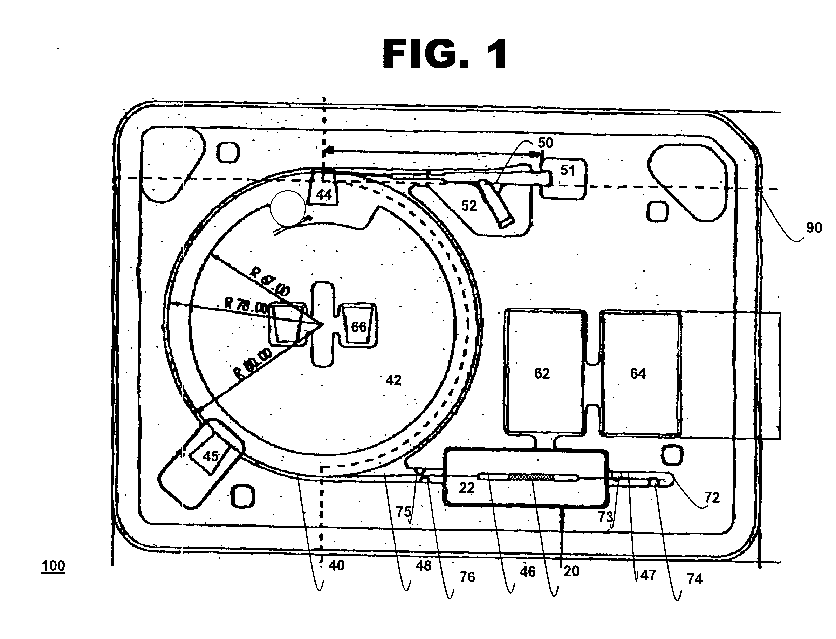

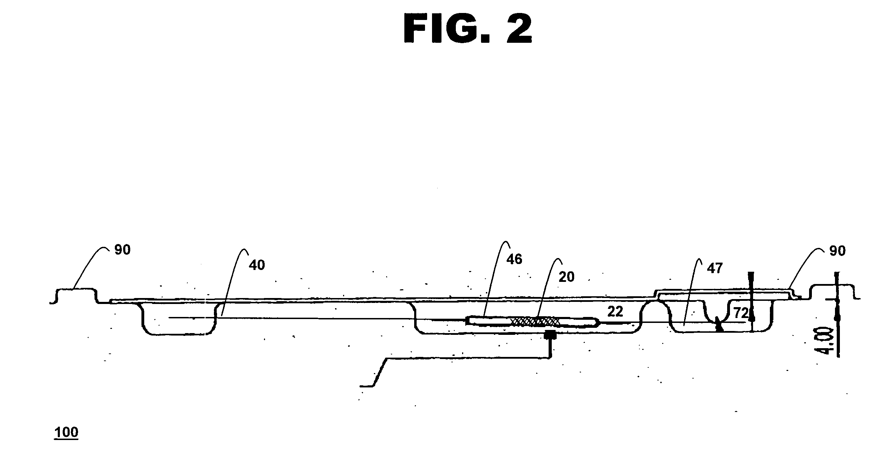

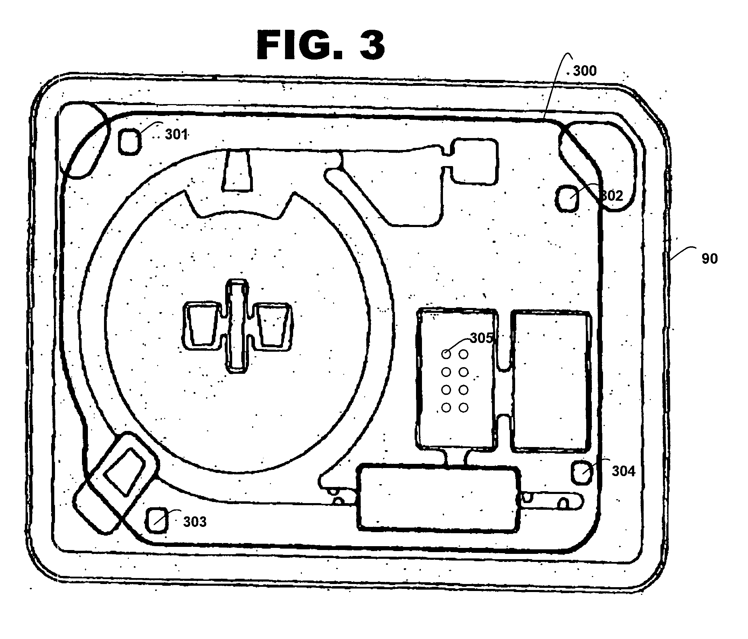

**FIG.** 1 shows one embodiment of a packaging system in accordance with the present invention at **100**. Packaging system **100** may include the packaging tray **90** of **FIG. 1** and may further include a lid **300** as seen in **FIGS. 2** and **3**. Packaging system **100** may comprise a stent **20** disposed within a void **22** of packaging tray **90**. As seen in **FIG. 2**, in one embodiment of the invention, a coating **30** may be dispersed on stent**20**. Packaging tray **90** may further comprise additional voids for other components of a stent assembly, including introducer void **52** in which an introducer assembly **50** is disposed. In the embodiment shown in **FIG. 1**, the stent **20** may be disposed upon a balloon catheter **40**. Balloon catheter **40** may further comprise an expandable balloon portion **46**. Packaging system **90** may further comprise a stylet **47**, which is removably positioned within stylet void **72**. Stylet **47** may be held in stylet void **72** by attachments **73**, **74** and stent **20** may be suspended upon stylet **47**. Thus, in one embodiment of the invention, stent **20** is suspended at two ends, using attachments **73**, **74** at one end and attachments **75**, **76** at the other end.

In one embodiment of the invention, a proximal portion**48** of balloon catheter **40,** which is proximal to stent **20** may be held fast by attachments **75, 76.** Because stent **20** is distally suspended on stylet **47** and the balloon catheter **40** on which stent **20** rests is proximally suspended at a portion **48**of balloon catheter **40,** stent **20** is suspended within void **22.** Stent **20** may thus be prevented from being jostled during shipment and storage. Damage of the coating **30** dispersed on the stent **20** may thus be avoided. All or part of the remaining proximal portion of balloon catheter **40** may be disposed in a catheter void **42** of packaging tray **90.** In the embodiment shown in **FIG. 1,** the shaft of balloon catheter **40** may be looped in tray **90** using one or more loop assemblies **44**, **45**. Packaging tray **90** may further comprise one or more voids **62**, **64**, **66** which may be used to hold other materials and accessories, including, but not limited to, dessicants, O₂ absorbers, other chemicals, extra looping assemblies and flushing cannulas.

In other embodiments of the invention, without a balloon catheter, stylet**47** may extend from stylet void **72** through void **22**. Stylet **47** may be held by attachments **73**, **74** at an end distal to stent **20** and held by attachments **75**, **76** at an end proximal to stent **20** and stent **20** may be suspended, without a catheter, on stylet **47**.

The stent void **22**, introducer void **52**, stylet void **72** and additional voids **62**, **64**, **66** of the present invention may be any suitable formation disposed within, formed as part of or attached to packaging tray **90** including but not limited to a well of packaging tray **90**, an indentation in packaging tray **90**, a space in packaging tray **90** or a hole in packaging tray **90**. Packaging system **100**may also comprise a plurality of voids of all shapes, sizes and formations. For example, packaging system **100** may comprise a stent void **22** that is an indentation formed within packaging tray **90** and an introducer void**52** that is a well attached to packaging tray **90**.

**FIG. 2** shows a cross-section of packaging system **100** As seen in **FIG. 2,** lid **300** may lie flat upon tray **90.** Stent **20** may be disposed within a void **22** of packaging tray **90**. In one embodiment of the invention, a coating **30** may be dispersed on stent **20**. Packaging tray **90** may further comprise additional voids for other components of a stent assembly, such as void **46**in which a loop assembly is disposed tor holding balloon catheter **40**, upon which stent **20** may be disposed. Balloon catheter **40** may further comprise an expandable balloon portion **45**. Packaging system **100** may further comprise a stylet **47**, which is removably positioned within void **72**. Stylet **47** may be held in stylet void **72** by attachments **73**, **74** and stent **20** may be suspended upon stylet**47**.

Stylet **47** may be any suitable assembly that enables suspension of stent **20** in void **22.** For example, stylet **47** may be an extended portion of balloon catheter **40** that is distal to stent **20.** Alternatively, stylet **47** may be an assembly that is insertable into the lumen of stent **20** and that may be attached with attachments **73**, **74** in such a manner as to suspend stent **20** in void **22**. For example, stylet **47** may be a thin needle or wire upon which stent**20** may be suspended. Alternatively, stylet **47** may be an assembly formed within tray **90**. For example, stylet **47** may be a thin column formed within void **22** that has an "eye" through which an end of catheter **40** may be threaded such that stent**20** is suspended within void **22**. Stylet **47** may be an assembly that is removable with stent **20** from tray **90**. For example, stylet **47** may be a modified extension of catheter **40**. Alternatively, stylet **47** may remain in tray **90** once **stent20** is removed.

In some embodiments of the invention, stylet void **72** of packaging tray **90** is sized and adapted so that all or a portion of stylet**47** may be held within void **72**. Stylet void **72** may be any suitably sized space or pocket for holding stylet **47**. For example, in one embodiment of the invention, stylet void **72** is a narrow channel into which stylet **47** protrudes. Stylet void **72** may be formed within packaging tray **90** using any suitable means, such as for example, thermomolding stylet void **72** within tray **90** when tray **90** is being thermoformed. Alternatively, tray **90** may be formed first and stylet void **72** inserted into the tray **90** afterwards.

Stylet void **72** may further comprise one or more attachment portions**73, 74.** These attachment portions **7374** attach stylet **47** within stylet void 72. As seen in **FIG. 1**, attachment portions **73**, **74** may be formed bumps along the sides of stylet void **72** that hold stylet **47** within stylet void **72**. Attachment portions **73**, **74** may be thermoformed within packaging tray **90**. Alternatively, attachment portions **73**, **74** may be inserted into tray **90** after the tray has been formed. For example, attachment portions **73, 74** may be adhesive segments that adhere to stylet **47** to hold it within stylet void **72**.

In some embodiments of the invention, void **22** of packaging tray **90** is sized and adapted so that all or a portion of stent **20** may be suspended within void **22**. Void **22** may be any suitably sized space or pocket in which a stent **20** may be suspended. For example, in one embodiment of the invention, void **22** is a well of packaging tray **90** that is 80mm in length by 27mm in width by 13mm in depth. Void **22** may be formed within packaging tray **90** using any suitable means, such as for example, thermomolding void **22** within tray**90** when tray **90** is being thermoformed. Alternatively, tray **90** may be formed first and void **22** inserted into the tray **90** afterwards.

Packaging tray **90** may further comprise additional voids for other components of a stent assembly, including introducer void **52** in which an introducer assembly **50** is disposed. In one embodiment of the invention, introducer void **52** is sized and adapted so that all or a portion of introducer assembly **50** may be placed into introducer void **52.** Alternatively, as seen in **FIG. 1**, packaging tray **90** may include a void **51** into which a portion of introducer assembly **50** may be introduced to suspend introducer assembly **50** in introducer void **52**. For example, a lure component of introducer assembly**50** may snap into void **51** to hold assembly **50** in place. Introducer void **52** may be any suitably sized space or pocket in which an introducer assembly**50** may be placed. For example, in one embodiment of the invention, introducer void **52** mimics the shape of an introducer assembly **50**. Introducer void **52** may be formed within packaging tray **90** using any suitable means, such as for example, thermomolding introducer void **52** within tray**90** when tray **90** is being thermoformed. Alternatively, tray **90** may be formed first and introducer void **52** inserted into the tray **90** afterwards.

Introducer assembly **50** may be used to introduce stent**20** via catheter **40** to the desired site. Introducer assembly **50** may be any suitable introducer assembly as is well known in the art. For example, introducer assembly**50** may be a conventional guide wire assembly.

Catheter **40** may be, for example, a low profile design with a tapered distal tip, and an inner lumen for insertion of a conventional guide wire. Any conventional or modified balloon catheter device may be used, such as a PTCA balloon catheter. In one embodiment of the invention, one or more guide wires may be stored in one or more of voids **62, 64, 66** of packaging tray **90**.

The expandable balloon portion **46**may be formed from a material such as polyethylene, polyehylene terephthalate (PET), or from nylon or the like. The length and diameter of the balloon may be selected to accommodate the particular configuration of the stent segment**20**. The balloon may be carried on any catheter, such as, for example PTCA low profile catheters and over the wire catheters.

In some embodiments of the invention, as described above, balloon catheter **40** may further comprise a stylet**47**, which protrudes into void **72**. Tip portion **47** of balloon catheter**40** may be held in stylet void **72** by attachments **73**, **74** while another portion **48** of balloon catheter**40** distal to stent **20** may be held fast by attachments **75, 76**. Because tip portion **47** is held by attachments **73, 74** and distal portion **48** is held by attachments **75, 76**, stent **20** is suspended within void **22**. Stent **20** may thus be prevented from moving and damage of the coating dispersed on the stent may be avoided. All or part of the remaining distal portion of balloon catheter**40** may be disposed in a catheter void **42** of packaging tray **90**. In the embodiment shown in **FIG. 1**, the shaft of balloon catheter **40** may be held in catheter void **42** of tray **90** using one or more loop assemblies **44, 45**.

In some embodiments of the invention, catheter void **42** of packaging tray **90** is sized and adapted so that all or a portion of catheter **40** may be contained within. Catheter void **42** may be any suitably sized space or pocket for containing a catheter**40**. For example, in one embodiment of the invention, void **42** is a circular well of packaging tray **90**. Catheter void **42** may be formed within packaging tray **90** using any suitable means, such as for example, thermomolding void **42** within tray **90** when tray **90** is being thermoformed. Alternatively, tray **90** may be formed first and catheter void **42** inserted into the tray **90** afterwards.

Catheter void **42** may further comprise one or more attachment portions **75, 76**. These attachment portions **75, 76** attach all or a portion of catheter**40** within catheter void **42**. As seen in **FIG. 1**, attachment portions **75, 76** may be formed bumps along the walls of catheter void **42** that hold catheter **40** within catheter void **42**. Attachment portions **75, 76** may be thermoformed within packaging tray **90**. Alternatively, attachment portions **75, 76** may be inserted into tray **90** after the tray has been formed. For example, attachment portions**75**, **76** may be adhesive segments that adhere to catheter **40** to hold it within catheter void **42**.

In one embodiment of the invention, tray **90** is fabricated of clear material to allow for viewing. Alternatively, the tray **90** may be fabricated of opaque material to aid in preventing degradation of the coating on stent**20** from light. The tray **90** may be made, for example, of a high barrier plastic, or other suitable materials. Tray **90** may be thermoformed, for example, from polyethylene, or another suitable material.

Packaging tray **90** may further comprise one or more voids **62, 64,66** which may be used to hold other materials and accessories. For example, a coated stent may be more sensitive to moisture. One or more of voids **62, 64, 66** may therefore be used to hold desiccant packets or oxygen absorber packets. Voids **62, 64, 66** may also hold any suitable chemicals that may be used to prolong shelf-life or prevent decay of coating **30** on stent **20**. Additional accessories may also be stored within one or more of voids **62**, **64**, **66**including, but not limited to, additional catheters, additional guidewires, additional introducer assemblies, flushing cannulas and additional stylets.

In some embodiments of the invention, voids **62, 64, 66** of packaging tray **90** are of varying sizes depending on what may be contained within each void. Each void **62, 64, 66** may also be individually adapted depending on its contents. Voids **62, 64, 66** may be formed within packaging tray **90** using any suitable means, such as for example, thermomolding voids **62, 64, 66** within tray**90** when tray **90** is being thermoformed. Alternatively, tray **90** may be formed first and voids **62, 64, 66** inserted into the tray **90** afterwards.

**FIG. 3** shows lid **300 of** packaging system **100.** In one embodiment of the invention, lid **300** is fabricated of clear material to allow for viewing. Alternatively, the lid **300** may be fabricated of opaque material to aid in preventing degradation of the coating **30** on stent **20** from light. The lid **300** may be made, for example, of a high barrier plastic, or other suitable materials. In some embodiments of the invention, tray **90** and lid **300** may be made of different materials. For example, tray **90** may be made of a high barrier plastic and lid **300**may be a foil cover over tray **90** or a foil pouch into which tray **90** may be sealed. Lid **300** may be made of foil, for example, in order to prevent moisture and oxygen from affecting components packaged in tray**90**. Alternatively, lid **300** may be made from the same material as tray **90.** For example, lid **300** and tray **90** may be formed of the same thermoformed plastic, such as polyethylene. In the embodiment of**FIG. 3**, lid **300** may fit into one or more indentations **301, 302, 303, 304.** Lid **300** may be raised over void **22** that contains coated stent **20**. Thus, lid **300** is prevented from contacting the coating **30** of stent **20**. Lid **300** may further comprise one or more openings **305.** Openings **305** may allow air circulation within packaging system **100.**

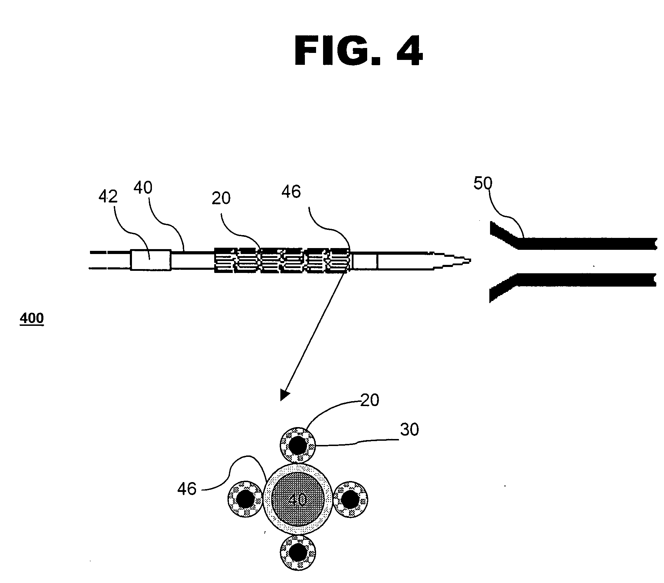

**FIG. 4** shows one embodiment of a coated stent for use with packaging system **100** at **400.** In the embodiment shown in **FIG. 4,** one stent segment**20** is shown. However more stent segments **20** may be used depending upon the size and configuration of the narrowed vessel to be treated. Additionally, when more than one stent segment **20** is used, the segments may be connected together by articulated or rigid joints, or multiple single stent segments may be deployed on the balloon catheter **20**. When more than one stent segmert **20** is deployed on the catheter **40**, each segment may have an associated void**22**. Alternatively, a plurality of stent segments **20** may be disposed within void **22**.

Stent segment **20** may be any suitable device for mechanically keeping an effective blood vessel open after completion of the angioplasty procedure. Such mechanical endoprosthetic devices, which are generally referred to as stents, are typically inserted into the vessel, positioned across the lesion, and then expanded to keep the passageway clear.Stent **20** may be for example, any stent known in the art, including, but not limited to, a coronary stent such as that sold by Medtronic as the S7 system. For example, stent segment **20** may be a self-expanding and expandable stent as is known in the art. Stent segment **20**may be a tubular slotted stents.

As seen in **FIG. 4**, some embodiments of the invention may include stent retainer rings **42** at one or both ends of the stent**20** to help to maintain the stent on the balloon. These retainers **42** may be located at the proximal and/or distal end of the balloon. Such retainers may be located on top of the balloon **46** or within the balloon **46.** Additionally, the balloon portion **46** itself may be used to form one or more stent retainers during encapsulation. Retaines **42** may assist in delivery by providing a smooth transition between the encapsulated stent and the catheter surface. In some embodiments of the invention, retainer rings **42** may interface with stent void **22** in order to hold stent **20** within the void **22**.

As seen in **FIG. 4**, coating **30** may comprise any suitable therapeutic agent for delivering therapy to a target site and/or any suitable substance within which such therapeutic agents may be dispersed. Coating **30**may be a coating adapted to deliver sustained release of therapeutic agent to target cells. Coating **30** may be, for example a biodegradable coating or a porous nonbiodegradable coating, having dispersed therein a sustained-release dosage form of one or more therapeutic agents as described below. Inan alternative embodiment, a biodegradable stent may also have the therapeutic agent impregnated therein, i.e., within the stent matrix of stent segment **20**. In yet another embodiment of the invention, the therapeutic agent(s) may be impregnated within stent segment **20,** which is further coated with a coating **30** having the sustained release-dosage form dispersed therein, is also contemplated. This embodiment of the invention would provide a differential release rate of the therapeutic agent, i.e., there would be a faster release of the therapeutic agent from the coating **30** followed by delayed release of the therapeutic agent that was impregnated in the stent matrix upon degradation of the stent matrix. The stent segment **20** may thus provide a mechanical means of increasing luminal area of a vessel, in addition to providing biological stenting action from the therapeutic agents releasably embedded therein.

Coating **30** may take the form of, for example non-degradable microparticulates or nanoparticulates or bbdegradable microparticulates or nanoparticulates. The microparticles or nanoparticles may be formed of a polymer-containing matrix that biodegrades by random, nonenzymatic, hydrolytic scission. One embodiment of coating **30** is formed of a mixture of thermoplastic polyesters (e.g., polylactide or polyglycolide) or a copolymer of lactide and glycolide components. The lactide/glycolide structure has the added advantage that biodegradation thereof forms lactic acid and glycolic acid, both normal metabolic products of mammals.

Coating 30 may also be, or may comprise a therapeutic substance which inhibits cellular activity at a target site in order to reduce, delay, or eliminate stenosis after angioplasty or other vascular surgical procedures. Coating **30**may also be a conjugate of several therapeutic substances. For example, coating**30** may comprise therapeutic agents that alter cellular metabolism or are inhibitors of protein synthesis, cellular proliferation, or cell migration; therapeutic agents that affect morphology or increases in cell volume; and/or therapeutic agents that inhibit extracellular matrix synthesis or secretion.

In one embodiment, coating **30** may include a non-cytotoxic therapeutic agent such as, for example, an antisense compound. One example of a non-cytotoxic therapeutic agent is NeuGene® antisense compound, Resten-NG (AVI-4126). Such antisense compounds compete at the mRNA level to block transcription of proteins that are involved in proliferation of the cells that cause restenosis. Antisense compounds may significantly reduce restenosis without prolonging healing times.

In one embodiment, coating **30** may include a cytotoxic therapeutic agent that is a sesquiterpenoid mycotoxin such as a verrucarin or a roridin. Coating **30** may also comprise cytostatic therapeutic agents that inhibit DNA synthesis and proliferation at doses that have a minimal effect on protein synthesis such as protein kinase inhibitors (e.g., staurosporin), suramin, and nitric oxide releasing compounds (e.g., nitroglycerin) or analogs or functional equivalents thereof. In addition, coating **30** may also comprise therapeutic agents that inhibit the contraction or migration of smooth muscle cells and maintain an enlarged luminal area following, for example, angioplasty trauma (e.g., the cytochalasins, such as cytochalasin B, cytochalasin C, cytochalasin D or the like). Coating **30**may also comprise vascular smooth muscle binding proteins that specifically associate with a chondroitin sulfate proteoglycan (CSPG) expressed on the membranes of a vascular smooth muscle cell.

In one embodiment of the invention, coating **30** may comprise agents that exhibit inhibition of a therapeutically significant target cell activity without killing the target cell, or target cell killing activity. For treatment of restenosis of vascular smooth muscle cells, useful therapeutic agents inhibit target cell activity (e.g., proliferation or migration) without killing the target cells. Example therapeutic moieties for this purpose are protein kinase inhibitors (e.g., staurosporin or the like), smooth muscle migration and/or contraction inhibitors (e.g., the cytochalasins, such as cytochalasin B, cytochalasin C, cytochalasin D or the like), suramin, and nitric oxide-releasing compounds, such as nitroglycerin, or analogs or functional equivalents thereof. In cancer therapy, useful therapeutic agents inhibit proliferation or are cytotoxic to the target cells. Example therapeutic moieties for this purpose are Roridin A and Pseudomonas exotoxin, or analogs or functional equivalents thereof. For treatment of immune system-modulated diseases, such as arthritis, useful therapeutic agents deliver cytostatic, cytocidal or metabolism-modulating therapeutic agents to target cells that are accessible by local administration of the dosage form. Example therapeutic moieties for this purpose are Roridin A, Pseudomonas exotoxin, suramin and protein kinase inhibitors (e.g., staurosporin), sphingosine, or analogs or functional equivalents thereof. For treatment of pathologically proliferating normal tissues (e.g., proliferative vitreoretinopathy, corneal pannus and the like), anti-proliferative agents or antimigration agents may be used (e.g., cytochalasins, taxol, somatostatin, somatostatin analogs, N-ethylmaleimide antisense oligonucleotides and the like).

Other examples of therapeutic agents that may be used alone or in combination within coating **30** include thrombin inhibitors, antithrombogenic agents, thrombolytic agents, fibrinolytic agents, vasospasm inhibibrs, calcium channel blockers, vasodilators, antihypertensive agents, antimicrobial agents, antibiotics, inhibitors of surface glycoprotein receptors, antiplatelet agents, antimitotics, microtubule inhibitors, anti-secretory agents, actin inhibitors, remodeling inhibitors, antisense nucleotides, anti metabolites, antiproliferatives, anticancer chemotherapeutic agents, antiinflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, inhibitors, free radical scavengers, chelators, antioxidants, antipolymerases, antiviral agents, photodynamic therapy agents, and gene therapy agents.

The dosage of therapeutic agents may be varied depending on the body lumen involved, the result desired, and the therapy indicated. Preferable therapeutic agents are dispersed within the microparticulates or nanoparticulates of coating **30**.

The dosage forms of coating **30** may be targeted to a relevant target cell population by a binding protein or peptide. These binding proteins/peptides may be, for example vascular smooth muscle cell binding protein, tumor cell binding protein and immune system effector oell binding protein. Other possible binding peptides include those that localize to intercellular stroma and matrix located between and among vascular smooth muscle cells. Peptides of this type are specifically associated with collagen, reticulum fibers or other intercellular matrix compounds. Tumor cell binding proteins associated with surface cell markers expressed by the target tumor cell population or cytoplasmic epitopes thereof may also be targeted by the present invention. Immune system-modulated target cell binding proteins associated with cell surface markers of the target immune system effector cells or cytoplasmic epitopes thereof may also be targeted with the present invention. The present invention may also be targeted to pathologically proliferating normal tissues.

Once the components held within packaging system **100**are removed from tray **90**, they may be used in any suitable manner known in the art. For example, stent **20** may be delivered to a desired treatment site with or without a guiding catheter and using a conventional guidewire for steerability to negotiate the area to be treated. Conventional radiopaque markers and fluoroscopy may be used with the device for positioning the stent and for viewing the expansion procedure. Once the stent is in place at the treatment site, the balloon portion **46** may be inflated in a conventional manner.

While the primary application for the stent is presently believed to be treatment of cardiovascular disease such as atherosclerosis or other forms of coronary narrowing, the stent assembly of the present invention may also be used for treatment of vessels in the kidney, leg, carotid, or elsewhere in the body. In such other vessels, the size of the stent may need to be adjusted to compensate for the differing sizes of the vessel to be treated.

## Claims

1. A coated stent with protective packaging, comprising:
at least one stent segment (20); and
a tray (90), including a stent void (22) disposed therein, wherein the stent segment is restricted from movement with respect to the tray while disposed within the stent void.

2. The stent of claim 1, further comprising:
a stylet (47) disposed within the tray (90) proximate the stent void (22) to suspend the at least one stent segment within the stent void.

3. The stent of claim 1, further comprising:
at least one first attachment (73) disposed within the tray (90) proximate the stent void (22) and distal to the stent void, wherein the first attachment retains the stent segment within the stent void.

4. The stent of claim 3, further comprising:
at least one second attachment (75) disposed within the tray (90) proximate the stent void (22) and proximal to the stent void, wherein the second attachment retains the stent segment within the stent void.

5. The stent of any preceding claim, further comprising:
a coating (30) dispersed on the at least one stent segment (20).

6. The stent of claim 5, wherein the coating (30) is selected from the group consisting of:
thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, artiplatelet agents, antimitotics, microtubule inhibitors, anti secretory agents, actin inhibitors, remodeling inhibitors, antisense nucleotides, anti metabolites, antiproliferatives, anticancer chemotherapeutic agents, anti-inflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, inhibitors, free radical scavengers, chelators, antioxidants, antipolymerases, antiviral agents, photodynamic therapy agents, gene therapy agents, and conjugates thereof.

7. The stent of any preceding claim, further comprising:
a catheter (40) attached to the stent segment (20).

8. The stent of claim 7, further comprising:
an expandable balloon portion (46) attached to the balloon catheter (40), wherein the expandable balloon portion expands an inner lumen of the stent segment.

9. The stent of claim 7, further comprising:
a catheter void (42) disposed within the tray (90), wherein the catheter void holds the catheter.

10. The stent of claim 9, further comprising:
at least one catheter attachment (75) disposed proximate the catheter void (42), wherein the catheter attachment retains the catheter within the catheter void.

11. The stent of claim 1, further comprising:
an introducer (50) to receive the stent segment (20).

12. The stent of claim 11, further comprising:
an introducer void (52) disposed within the tray (90) wherein the introducer void holds the introducer.

13. The stent of claim 1, further comprising:
at least one accessory void (62) disposed within the tray to hold an accessory.

14. The stent of claim 13 wherein the accessory is selected from the group consisting of:
desiccant packets, oxygen absorber packets, chemical packets, catheters, guidewires, cannulas and stylets.

15. The stent of any preceding claim, further comprising:
a lid (300), wherein the lid connects to the tray (90).

16. A system for treating heart disease, comprising:
a catheter (40);
a coated stent (20) with protective packaging as claimed in any of claims 1 to 6 coupled to the catheter, the stent including a drug polymer coating (30).

17. The system of claim 16, further comprising:
an expandable balloon portion (46) attached to the catheter (40).

18. The system of claim 16 or 17, further comprising:
a catheter void (42) to hold the catheter within the tray.

19. The system of claim 16, further comprising:
an introducer (50) to receive the stent segment (20).

20. The system of claim 16, further comprising:
an introducer void (52) to hold an introducer within the tray.

21. The system of claim 16, further comprising at least one accessory selected from the group consisting of:
desiccant packets, oxygen absorber packets, chemical packets, catheters, guidewires, cannulas and stylets.

22. The system of claim 16, further comprising:
at least one accessory void (62) to hold at least one accessory within the tray.

23. The system of claim 16, further comprising:
a lid (300) to seal the tray.

24. A method of protecting a coated stent, comprising:
providing a tray (90) including a stent void (22) therein; and
suspending the coated stent within the stent void such that the coated stent is restricted from movement with respect to the tray when placed in the stent void.
